# EUROPEAN PATENT APPLICATION

(11) **EP 4 047 097 A1**
(43) Date of publication of application: **24.08.2022**
(21) Application number: 20838333.1
(22) Date of filing: 14.10.2020
(51) Int. Cl.: C12P 19/04, C12N 1/20, C12R 1/21, A61K 39/102

(54) **METHOD FOR THE PRODUCTION, RECOVERY AND PURIFICATION OF CAPSULAR POLYSACCHARIDE POLYRIBOSYLRIBITOL PHOSPHATE (PRP) AND USE THEREOF**

(30) Priority: 14.10.2019 BR 102019021510
(71) Applicant: INSTITUTO BUTANTAN, 05503900 São Paulo - SP (BR)
(72) Inventor: TAKAGI, Mickie, 05588-001 São Paulo - SP (BR); CABRERA-CRESPO, Joaquin, 05588-001 São Paulo - SP (BR); SIMAS, Rodrigo Gabriel, 05588-001 São Paulo - SP (BR); BRAGA, Luciano Gama, 05588-001 São Paulo - SP (BR); ALBANI, Silvia Maria Ferreira, 05588-001 São Paulo - SP (BR)
(74) Representative: Petraz, Gilberto Luigi
(86) International application number: PCT/BR2020/050410
(87) International publication number: WO 2021/072520

(57) **Abstract**

The present invention relates to methods involved in the production and purification of the capsular polysaccharide polyribosylribitol phosphate (PRP) produced by the bacteria Haemophilus influenza type b (Hib) to obtain the polysaccharide with suitable molecular mass and purity for subsequent formulation of the Hib conjugate vaccine. The present invention also relates to the use of the capsular polysaccharide polyribosylribitol phosphate (PRP) to prepare the vaccine.

## Description

### Field of the Invention

The present invention relates to a process for production and purification of the capsular polysaccharide Polyribosyl-Ribitol-Phosphate (PRP) produced by the bacterium *Haemophilus influenzae* type b (Hib). More specifically, the present invention relates to a purification process of polysaccharide (PRP) with purity and molecular weight suitable for the subsequent formulation of Hib conjugate vaccine.

### Background of the invention

*Haemophilus influenzae* are Gram-negative, aerobic/microaerophilic bacteria, having cocobacillary morphology, being immobile, not being sporulated, which are present in the nasopharynx of 75% of healthy children and adults, thus constituting asymptomatic carriers. There is no record of detection of this microorganism in any other animal species (ADA and ISAACS, 2003; KELLY et al., 2004).

It was isolated in 1890 by Pfeiffer during a flu pandemic, it being mistaken as the cause of the flu. *H. influenzae* was probably an important secondary invader to the influenza virus (influenza) in the pandemic of 1890 and in many others that followed (PITTMAN, 1931).

Since they are fastidious organisms, the genus *Haemophilus* ("blood friends") requires precursors of heme for growth, which are present in the blood, specifically factor X (ie, hemin) and factor V (NAD or NADP) (HARRISON et al., 2008) which are the factors responsible for growth. This bacterium better grows between 35 and 37°C, with an optimum pH around 7.6. In the laboratory, it grows in aerobic conditions, with low CO₂ tension (5% CO₂), although it is capable of making the glycolytic pathway and the respiratory chain using nitrate as the final electron acceptor.

In 1995, *H. influenzae* was the first organism to have the genome entirely sequenced, and the genetic information generated showed important characteristics about virulence factors and vaccine targets, as well as knowledge about the different metabolic routes of this microorganism (ALI et al., 2002).

A fraction of asymptomatic carriers, which corresponds to 3 to 7%, can harbor pathogenic variants of *Haemophilus influenzae,* it being of great importance in the transmission of the bacterium. Children under the age of five, the elderly and immunosuppressed individuals are the most affected by this infection, which includes meningitis, pneumonia, epiglottitis, septicemia, etc. (KELLY et al., 2004).

This bacterial species may have an external structure, called capsule. Depending on the composition of the polysaccharide that composes it, it is classified into six different serotypes (a, b, c, d, e, f). Uncapped ones are classified as non-typable (KELLY et al., 2004).

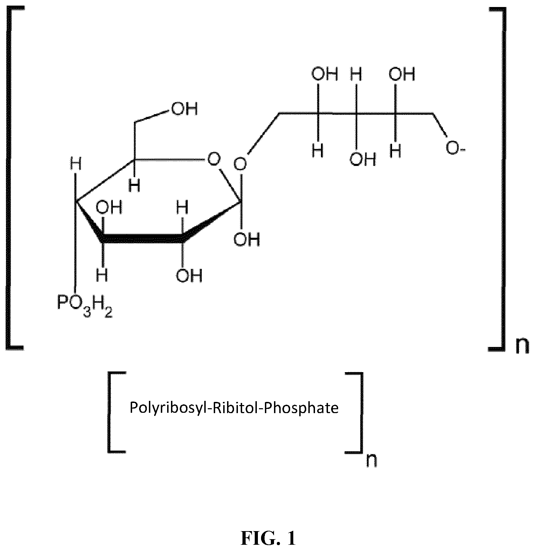

Among the infections produced by *Haemophilus,* 95% are caused by bacteria that have type b polysaccharide capsule. The pathogenesis of *H. influenzae* type b infection is not completely clear, although the presence of type b polysaccharide capsule, or Polyribosyl-Ribitol-Phosphate (PRP) is considered the main virulence factor, which is composed by monomers containing units of ribose, ribitol and phosphate (Figure 1) (CRISEL et al., 1975).

The capsular structure has anti-phagocytic properties and is unable to induce the alternative complement pathway, allowing the bacteria to invade the bloodstream and the cerebrospinal fluid, without attracting phagocytes or causing inflammatory responses and complement-mediated lysis. For this reason, antibodies against the capsule, which promote both phagocytosis and bacterial lysis, are the main factors of the immune defense against infection by *H. influenzae* type b (ADA and ISAACS, 2003; ST. GEME and CUTTER, 1996.

The effective way to prevent diseases caused by *H. influenzae* type b, especially among children under the age of five, is through public vaccination programs, which were implemented in Brazil in 1999 (KMETZSCH et al., 2003). Such a strategy generated positive impacts, decreasing incidence and mortality (MIRANZI et al., 2006).

The use of vaccines constituted by the bacterial capsule is only capable of inducing a strong primary antibody response, but with a small induction of immune memory. in order to circumvent this undesirable characteristic, conjugated vaccines have emerged, in which the Hib PRP is chemically conjugated to a protein of choice to modify the type of antigen presentation and the type of induced immune response, thus guaranteeing the immune memory response of the T-dependent type, which is efficient in the age group with the highest risk (KELLY et al., 2004).

Although conjugated vaccines are already produced and marketed by pharmaceutical multinationals, it is of national interest to produce them in Brazil, as these vaccines are very expensive and the country needs autonomy to develop such an important immunizing agent. Thus, the improvement in the conditions of production and purification of these microbial compounds remains to be important for preventive public health policies.

The production of vaccines is comprised of different processes, such as the cultivation of the microorganism in bioreactors, called production and subsequent inactivation of the same; followed by purification. In the particular case of conjugated vaccines, the final step corresponds to the process of chemical conjugation of the purified polysaccharide to a protein accompanied by the purification of the conjugated product.

The production of a given bioproduct, on a large scale, is carried out in bioreactors that have computerized systems capable of controlling aeration, agitation, pH, temperature; in addition to pumps for adding nutrients, acid, alkali, etc. (WARD, 1989; WARD, 1991; PACE, 1987; TAKAGI et al., 2003; TAKAGI et al., 2008).

In the particular case of Hib, the traditional culture medium BHI (Brain Heart Infusion) resulted in 91 mg/L of polysaccharide (CARTY et al., 1985). Anderson (1977) obtained the best results with the medium containing casamino acid and yeast extract buffered with 0.1 M potassium phosphate at pH 7.5, reaching 182 mg/L of polysaccharide. Following the WHO recommendations, a medium without animal components (animal free) was tested. The medium containing soy peptone and yeast extract MP increased PRP production to 258 mg/L, fed batch cultivation with Eagan strain and medium containing yeast casaminoacid-extract for the production of the capsular polysaccharide of *H. influenzae* b, was described by Merrit et al. (2000), thereby obtaining a polysaccharide concentration of 1,200 mg/L, much higher than the batch process in which the value of 490 mg/L was obtained and also those described in all patents.

Takagi et al. (2006) studied the effects of surface and submerged aeration, the concentration of dissolved oxygen controlled at 10 and 30% of air saturation, and the interference of pH control in cell growth and polysaccharide production. In the study, a culture medium containing soy peptone and yeast extract was used as complex medium ("animal free"). In these conditions, it was found that, by using surface aeration, 421 mg PRP/L were obtained, while under submerged aeration, at 10 and 30% of air saturation without pH control, 550 mg PRP/L were reached. However, the introduction of pH control was the great differential, generating 950 mg PRP/L. The maintenance of the dissolved oxygen concentration at 10 or 30% of the air saturation did not result in significant differences in the polysaccharide formation. Cultivations carried out in fed batches, with the same strain and culture medium, with pH control and submerged aeration at 30% air saturation, using feed medium containing yeast glucose-extract in the ratio (C: N) of 2:1, presented a final polysaccharide value of 2,000 mg PRP/L (TAKAGI et al., 2007).

According to Takagi et al. (2006), the improvement in PRP productivity is due to three important changes in the cultivation protocol, which depends on the composition of the medium, aeration and pH control at 7.2. After several studies, it was observed that the production of PRP is not directly related to dissolved oxygen, since pH can, in fact, influence, affecting the metabolic route, causing changes in the composition and size of PRP, considering that the same is under the control of several genes (TAKAGI et al., 2006).

PRP synthesis is one of the limiting factors in the production of polysaccharide vaccines, which is in the order of 0.25-2 g/L of culture. Unlike proteins, it is dependent on several genes and, in most cases, the metabolic pathway thereof is not yet easily manipulated (BENTLEY et al., 2006; TAKAGI, 2003).

After the completion of the cultivation, the next step corresponds to purification (downstream process), in which the "crude" product is processed until the required degree of purity is obtained for the use thereof, seeking to maximize its recovery and minimize the cost (WARD, 1991; KASTNER and GOLKER, 1987).

Purification involves a series of independent steps, in which the different physico-chemical properties of the product of interest and contaminants are used, so that, progressively, the fraction of interest is enriched, and the unwanted components are eliminated. The purification step is based on the different properties of the molecules, such as solubility, hydrophobicity, electrical charge, molecular size, special properties (thermoresistance), affinity for other molecules; besides the cellular localization (intra or extracellular) (WARD, 1989; WARD, 1991; SOFER and HAGEL, 1997; ANSEJO and PATRICK, 1990).

In addition to seeking for achieving relative purity, biotechnological products must comply with standards and requirements regarding the allowed residual amount of impurities, such as nucleic acids, proteins and pyrogenic substances, limits related to "color", absence of enzymatic activities harmful to product and which may compromise the use (e.g.: proteases, hemolytic activity). The standards to be met are those of regulatory agencies, such as ANVISA (National Health Surveillance Agency), FDA (Food and Drug Administration), WHO (World Health Organization) or pharmacopeias. The complex process of purifying these products represents 20 to 80% of the total cost (SCOPES, 1994; ERSSON et al., 1998; SOFER and HAGEL, 1997).

The traditional polysaccharide purification method is characterized by several ethanol precipitation steps, extraction by organic solvents, such as phenol, and centrifugation/ultracentrifugation steps. However, the use of ethanol implies the use of a continuous centrifuge at high speeds and longer times, since the "pellet" often does not have a consistent characteristic. In addition, the physical space must be appropriate, with explosion-proof installations, and handling care, which requires cost and time.

Patent application BR 10 2014 021245, filed on August 27, 2014, published on March 8, 2016, in the name of MSD WELLCOME TRUST HILLEMAN LABORATORIES PVT. LTD and entitled: "QUICK PROCESS TO PRODUCE AND PURIFY HIB-PRP" describes a fast process to produce and purify the PRP polysaccharide from *Haemophilus influenzae* type b which meets WHO specifications. The process produces PRP polysaccharides capable of being used as such, or of being derivatized or linked to other molecules to produce a vaccine against infections by *Haemophilus influenzae* type b. The process describes a simpler, faster, more cost-effective and scalable method, in which the PRP is purified in a significantly reduced time at room temperature.

Although the patent application BR 10 2014 021245 discloses a process for producing and purifying PRP, this process has different technical characteristics from the process for producing and purifying PRP of the present invention, such as the CTAB (hexadecyltrimethylammonium bromide) cationic detergent is not used and the centrifugation steps were replaced by tangential flow microfiltration.

Patent application BR 11 2014 033082, filed on July 2, 2013, published on June 27, 2017; in the name of SANOFI PASTEUR and entitled: "PROCESS OF PRODUCTION OF ANTIGENS *HAEMOPHILUS INFLUENZAE* TYPE B" describes a process for the production, on an industrial scale, of capsular polysaccharide of *Haemophilus influenzae* type b (PRP) for vaccine purposes, according to which a strain of *Haemophilus influenzae* type b (Hib) is cultivated, in a culture medium, collecting the culture supernatant to extract the capsular polysaccharide, that culture medium comprising at least: a carbon source, protoporphyrin, salts, amino acids, NAD or NADH, vitamins, pH regulation medium, characterized in that this culture medium is chemically defined.

The process disclosed by patent application BR 11 2014 033082 is a process in which the culture medium of a Hib strain is characterized, which is a chemically defined medium, whose nature and amount of each of the components is perfectly defined. Patent application BR 11 2014 033082 does not describe, not even suggests, a process for producing and purifying PRP, such as the process described by the present invention.

US patent 4,220,717, filed on September 2, 1980, in the name of AMERICAN CYANAMID CO and entitled "ISOLATION AND PURIFICATION OF POLYRIBOSYL RIBITOL PHOSPHATE FROM *HAEMOPHILUS INFLUENZAE* TYPE B" describes a process for the isolation and purification of the PRP immunologically active, the capsular polysaccharide of *Haemophilus influenzae* type b. The PRP was purified with ethanol, CTAB and a phosphate-containing adsorbent, hydroxylapatite [3.Ca₃(PO₄)₂.Ca(OH)₂]. Contaminants (nucleic acid, proteins and endotoxins) are removed to the minimum level by hydroxyapatite treatment. Also described is a process for the preparation of a combined vaccine containing *H. influenzae* and *B. pertussis* antigens. The vaccine causes formation of anti-PRP antibodies and anti-pertussis antibody (measured by microagglutination) in young animals. This serum with anti-PRP antibody shows strong bactericidal activity.

Although US patent 4,220,717 discloses a process for producing and purifying PRP, this process has different technical characteristics from the process for producing and purifying PRP disclosed by the present invention, such as in the search object, CTAB is not used for removal contaminants, as well as hydroxylapatite is not used for adsorption separation.

US patent 7,582,459, filed on September 10, 2004, published on March 22, 2007, in the name of NL VACCININST and entitled: "PROCESS FOR PRODUCING A CAPSULAR POLYSACCHARIDE FOR USE IN CONJUGATE VACCINES" describes a method to produce a polysaccharide and a conjugate vaccine, including the polysaccharide produced according to the method. A characteristic step in the method is that the pH of the culture medium is maintained at a constant value with base or acid until adjustment with base or acid, respectively, is no longer possible.

Although US patent 7,582,459 discloses a method for producing a bacterial capsular polysaccharide, this process has different technical characteristics from the process for producing and purifying PRP of the present invention, such as in the search object CTAB cationic detergent is not used, as well as the centrifugation steps were replaced by tangential flow microfiltration.

US patent 4,196,192, filed on October 28, 1977, published on April 1, 1980, in the name of AMERICAN CYANAMID and entitled: "COMBINED *HAEMOPHILUS INFLUENZAE* TYPE B AND *PERTUSSIS* VACCINE" describes a process for isolation and purification of immunologically active polyribosyl-ribitol phosphate (PRP), the capsular polysaccharide of *Haemophilus influenzae* type b. The PRP was purified with ethanol, CTAB and a hydroxylapatite adsorbent. Contaminants (nucleic acid, proteins and endotoxins) are removed to the minimum level by hydroxyapatite treatment. Also described is a process for preparing a combined vaccine containing the PRP antigens (prepared as mentioned above) and *B. pertussis.* The vaccine causes formation of anti-PRP antibodies and anti*-pertussis* antibody (measured by microagglutination) in young animals. This serum with anti-PRP antibody exhibits strong bactericidal activity.

Although US patent 4,196,192 discloses a method for producing a bacterial capsular polysaccharide, this process has different technical characteristics from the process for producing and purifying PRP of the present invention, as in the present invention no cationic detergent is used, as well as the centrifugation steps were replaced by tangential flow microfiltration. In addition, in the present invention, CTAB is not used to remove contaminants, nor hydroxylapatite for adsorption separation.

The Dissertation on behalf of SILVIA MARIA FERREIRA ALBANI presented to the Post-Graduate Program in Inter-Units in Biotechnology USP / Institute Butantan / IPT, entitled "ALTERNATIVE METHODS FOR PURIFICATION OF THE CAPSULAR POLYSACARIDE OF *HAEMOPHILUS INFLUENZAE* TIPO, B", in 2008, refers to the bibliographic review carried out by the author synthetically describing the method for the purification of PRP, where it is described the classic process of purification of the PRP involving several steps of precipitation with ethanol and cationic detergent (flammable and polluting), extraction with phenol (toxic and corrosive) and centrifugation / ultracentrifugation steps.

In this Dissertation document, the author concludes that the best way to recover the PRP is through centrifugation. Furthermore, in this document, unit operations of tangential microfiltration as disclosed by the present invention were not employed.

In the process disclosed by the dissertation document on behalf of SILVIA MARIA FERREIRA ALBANI, two steps of 0.22 µm tangential microfiltration are employed. In the first of them the solvent used is 30% ethanol, while in the second step the solvent is 80% ethanol. These ethanol concentrations were also used in two steps of precipitation. This method is different from the present invention, in that there is only tangential microfiltration in the presence of ethanol (40%) and the ethanol concentrations used in the precipitation steps are different, namely, 40 and 60%. Changing the ethanol concentration is not an obvious solution for improving the process presented in that dissertation in the name of SILVIA MARIA FERREIRA ALBANI.

The publication in the name of Silvia Maria Ferreira Albani et al. and entitled: "POLYSACCHARIDE PURIFICATION FROM HAEMOPHILUS INFLUENZAE TYPE B THROUGH TANGENTIAL MICROFILTRATION", published in Carbohydrate Polymers 116 (2015) pages 67-73 on March 28, 2014 reveals a process for purifying PRP produced by *Haemophilus influenzae* type b, developed from the reference process presented in document D6. However, except for cell separation, the centrifugation steps were replaced by tangential microfiltration.

In contrast to the present invention, the process presented in the publication in the name of Silvia Maria Ferreira Albani et al. does not use tangential microfiltration for cell separation, where this step is essential in obtaining the desired PRP purity in the present invention.

### Summary of the Invention

in order to solve the problems mentioned above, the present invention will provide significant advantages in relation to the production, recovery and purification processes of PRP produced by the bacterium *Haemophilus influenzae* type b, enabling an increase in the performance thereof and presenting a more favorable cost/benefit ratio.

The present invention relates to processes involved in the production and purification of the capsular polysaccharide Polyribosyl-Ribitol-Phosphate (PRP) produced by the bacterium *Haemophilus influenzae* type b (Hib).

The objective of the present invention is to obtain the polysaccharide having purity and adequate molecular mass for the subsequent formulation of Hib conjugate vaccine. A possible final presentation would be the pentavalent vaccine, which provides resistance to five diseases: DTP (diphtheria-tetanus-pertussis), HepB (hepatitis B) and Hib.

The process disclosed by the present invention comprises two distinct steps: (a) production of the polysaccharide by fermentation and (b) recovery and purification of the polysaccharide.

The process disclosed by the present invention involves the use of complex medium of vegetable origin in substitution to complex medium of animal origin used in the state of the art. The preparation of the seed batch and work batch was adopted to maintain homogeneity between the batches of tests performed, allowing the monitoring of cellular viability as a criterion for batch validity. Inoculum and pre-inoculum were performed in a liquid medium instead of plating to reduce the process time. The cultivation is carried out in a batch regime fed at constant flow, lasting less than 24 hours. The concentration of PRP at the end of the cultivation reaches values above 1400 mg/L, much higher than the concentrations reached by the processes disclosed in the state of the art, below 500 mg/L. Regarding the polysaccharide purification, this is an innovative process, as there are significant changes in relation to the processes described in the state of the art.

The processes described in the state of the art use CTAB and/or deoxycholate detergents - DOC (animal origin) and are mainly based on the precipitation of PRP or impurities with ethanol. The process disclosed by the present invention also uses ethanol, but at different steps in the process.

The use of DOC and CTAB detergents was also avoided in the present invention. In addition, the centrifugation steps have been replaced by tangential flow microfiltration.

The process of the present invention has a lower number of steps than the state of the art processes, thus, the consumption of reagents is very low, due to the change in the concentrations of ethanol used in each step.

The process of the present invention proved to be scalable, which renders possible to perform it on a pilot scale.

### Brief Description of the Drawings

The structure and operation of the present invention, together with additional advantages of the same can be better understood by reference to the attached drawings and the following description:
Figure 1 shows the chemical structure of the polysaccharide capsule (PRP) of *H. influenzae* type b;
Figure 2 refers to the cultivation process of *Haemophilus influenzae* type b for the production of PRP according to an embodiment of the present invention;
Figure 3 shows the flowchart of the complete process of clarification of the fermented broth by Hib and purification of the PRP of the present invention;
Figure 4 shows the influence of the cell separation strategy on the recovery and purity of the PRP. In the graphic, a process with cell separation by centrifugation and two different processes by tangential microfiltration (MF (A) and MF (B)) are compared;
Figure 5 shows the relative purity of PRP in relation to proteins (PR Prot = gPRP/gProtein) and nucleic acids (PR AN = gPRP/gnucleic acids) in the supernatant fraction after precipitation of the fermented broth with 30% ethanol in presence of selected surfactants. In this additive selection experiment, the ethanol concentration used was still 30%;
Figure 6 shows the effect of adding different concentrations of SDS (sodium dodecyl sulfate) on the recovery of PRP in step [2MF]. In this case, the concentration of ethanol used corresponds to the optimized condition of the process. The combination of 0.5% SDS and 40% ethanol resulted in a significant improvement in PRP recovery during the tangential microfiltration step [2MF];
Figure 7 shows the precipitation profile of ethanol-purified PRP in the presence of different chlorine salts. Saline concentration equal to 200 mM;
Figure 8 shows the effect of sodium acetate and ethanol concentrations on PRP recovery after the second precipitation step;
Figure 9 shows PRprot (dark gray) and PRAN (light gray) in the purification of different PRP batches. The black lines crossing the bars indicate the respective values found in the control. Control condition: 30% ethanol and 5% sodium acetate in the first precipitation; 80% ethanol and 7% sodium acetate in the second precipitation. Optimized condition: 40% ethanol in the first precipitation; 60% ethanol and 7% sodium acetate in the second precipitation;
Figure 10 shows the scheduling problems. Tangential ultrafiltration [2UF] in 100 kDa pore membrane. 40% solvent ethanol in water;
Figure 11 shows the effect of ethanol concentration in the second step of tangential ultrafiltration in a 100 kDa pore membrane. 0.5 m² membrane; and
Figure 12 shows the effect of salt concentration on the aqueous fraction of the 40% Ethanol solution in the second microfiltration step of the PRP purification process.

### Detailed Description of the Invention

Although the present invention may be susceptible to different modalities, it is shown in the drawings and in the following detailed discussion, a preferred embodiment with the understanding that the present embodiment should be considered as an example of the principles of the invention and is not intended to limit the present invention to what has been illustrated and described herein.

The present invention relates to a process of production and purification of the capsular polysaccharide Polyribosyl-Ribitol-Phosphate (PRP) produced by the bacterium *Haemophilus influenzae* type b (Hib). More specifically, the present invention relates to a process of production and purification of the polysaccharide (PRP) with purity and molecular mass suitable for subsequent chemical conjugation and result in the Hib conjugate vaccine.

Figure 1 shows the chemical structure of the polysaccharide capsule (PRP) of *H. influenzae* type b.

### 1. Cultivation of Haemophilus influenzae type b

Initially, storage and working batches are prepared from lyophilisate of the strain of *Haemophilus influenzae* type b GB3291, producer of the capsular polysaccharide Poliribosil-Ribitol-Phosphate (PRP), acquired from the Culture and Microorganism Collections Center - *Instituto Adolfo Lutz* - SP with purity assessment by Gram method and visual determination of cellular viability.

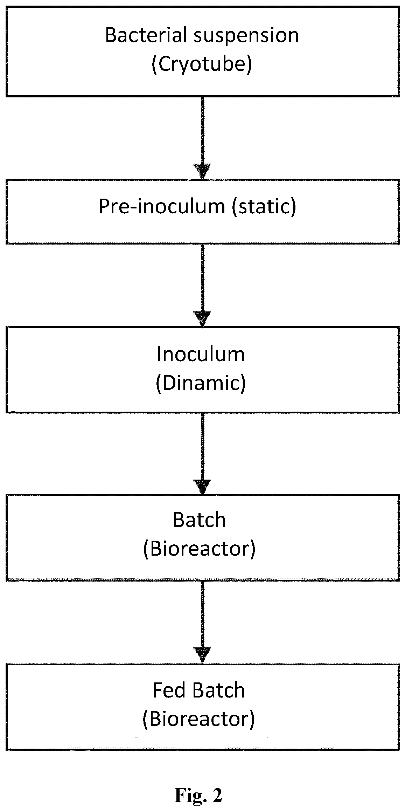

The bacterial suspension is distributed in 1 mL cryovials, frozen at -20°C for 12 hours and then maintained until the moment of use in liquid nitrogen. The production of PRP occurs as outlined in Figure 2 and detailed below.

The pre-inoculum is prepared from the bacterial suspension of the work lot stored in liquid nitrogen. A volume of 500 uL of the bacterial suspension is transferred to a 300 ml Erlenmeyer flask containing 50 ml of complex medium containing peptones of plant origin and yeast extract. The sample is kept static at 37°C for 7 - 10 h in an anaerobiosis chamber.

After that period, the optical density of the sample is measured at 540 nm (DO540) and transferred to the 2 L inoculum flask, in an adequate volume so that 400 mL of the resulting suspension has a DO540 equal to 0.025.

The suspension, referred to as inoculum, is stirred in an orbital shaker at 250 RPM and 37°C for at least 12 hours, until DO540 is reached between 5 and 7. Transfer the appropriate inoculum volume so that the initial DO540 of the culture medium (Table 1), present in the bioreactor, is 0.1.

Bacterial cultivation is carried out in a simple batch regime until total depletion of the glucose in the medium occurs. At that point, it is initiated the batch step fed by the addition of feed medium as shown in Table 1.

**Table 1 - Composition of culture and food media used in the cultivation of Haemophilus influenzae type b**

| | **Culture medium** | **Feeding medium** |
|---|---|---|
| **Soytone (g/L)** | **10.0** | **10.0** |
| **Yeast extract (g/L)** | **5.0** | **100.0** |
| **Glucose (g/L)** | **5.0** | **200.0** |
| **NaCl (g/L)** | **5.0** | **5.0** |
| **K₂HPO₄ (g/L)** | **2.5** | **2.5** |
| **Na₂HPO₄ (g/L)** | **10.5** | **10.5** |
| **NaH₂PO_{4*}H₂O (g/L)** | **5.8** | **5.8** |
| **NAD (g/L)** | **0.015** | **0,015** |
| **Hemine (g/L)** | **0.03** | **0.03** |

The process is completed after 20 hours of cultivation with DO540 around 20. After completion of the cultivation, sodium azide is added to the final concentration of 0.2% and then the polysaccharide purification process begins.

### 2. Clarification of the fermented broth and purification of the PRP

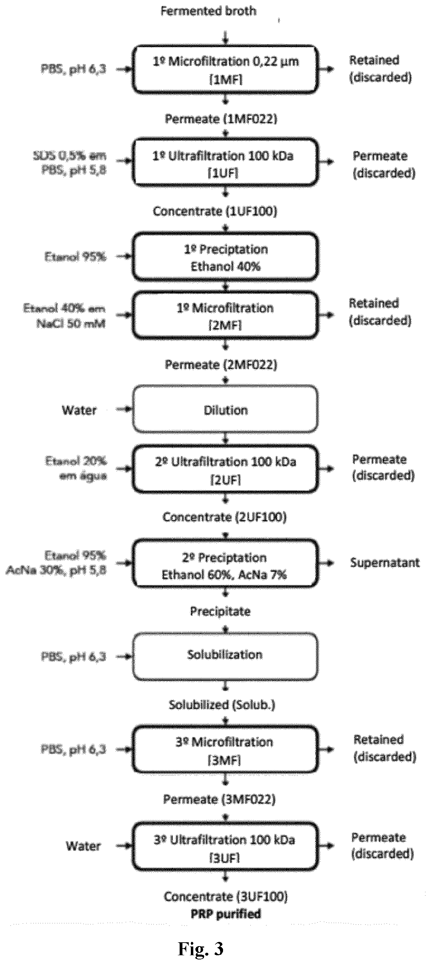

The complete process for the clarification and purification of PRP of the present invention is shown in Figure 3.

This process was developed with the objective of reducing the number of steps of the processes already established in the state of the art, in addition to replacing the unitary centrifugal operations by tangential flow filtration.

The process of the present invention can be grouped into three operating blocks, each with tangential flow microfiltration using 0.22 µm pore membranes followed by tangential flow ultrafiltration using nominal 100 kDa pore membranes.

The combination of micro and ultrafiltration makes the process compact in terms of space and manpower used, bearing in mind that these can be carried out concurrently, occupying the same environment and being controlled by the same operators.

The proposed sequence of steps, mainly ethanol precipitation, does not match the already established processes for the purification of PRP revealed in the state of the art. This sequence resulted in the removal of impurities in early steps of the process, which enabled the formulation of a process with a lower number of unit operations.

The product obtained showed high molecular mass (> 100 kDa) and purity in line with the requirements of international agencies. Below, each processing step will be presented in detail.

### 2.1 First microfiltration, [IMF]

The first microfiltration of the process of the present invention aims at the separation between bacterial cells and the PRP present in the extracellular medium.

This step is commonly referred to as clarification. Traditionally, the clarification of the fermentation broth is carried out by centrifugation, but in the process developed by the present invention, tangential flow microfiltration was used.

Microfiltration is performed with a membrane with a pore having a diameter of 0.22 µm. During this step the cells are dialyzed. The dialysis solution is PBS buffer (150 mM NaCl, 10 mM NaH₂PO₄Na₂HPO₄, pH 6.3). Dialysis is performed at constant volume with 15 - 25 diavolumes of buffer. In the permeate or microfiltered fraction (1MF022) it is contained the PRP. The cells are present in the fraction retained by the membrane, which is discarded.

### 2.2 First ultrafiltration, [1UF]

The fraction 1MF022 is subjected to concentration and dialysis by ultrafiltration using a nominal molecular cut membrane of 100 kDa. The content is initially concentrated 15 to 45 times and then dialyzed at constant volume with 6 diavolumes of PBS buffer plus 0.5% SDS (pH 5.8 - 6.3). The concentrated fraction is referred to as [1UF100]. The addition of SDS has not been reported in other patents. We verified the need for this detergent for the greatest removal of impurities during the first ultrafiltration, [1UF], and in the first precipitation step, as well as better performance of the second microfiltration step, [2MF].

### 2.3 First precipitation with 40% ethanol

The first precipitation with ethanol is performed by adding ethanol (95 - 99.5%) to the 1UF100 fraction. The final concentration of ethanol is 40% (v/v). The content is then kept under stirring for up to 16h at room temperature.

The objective of this step is the precipitation of impurities, mainly proteins and nucleic acids, while the PRP remains soluble. This is a significant difference from the state of the art, considering that in the state of the art the first precipitation has the objective of concentrating the PRP by precipitation. For this purpose, higher concentrations of ethanol are added, usually in combination with the CTAB detergent.

### 2.4 Second microfiltration, [2MF]

The precipitated content of the previous step is then subjected to microfiltration with a membrane having a pore diameter equal to 0.22 µm.

The objective is the retention of precipitated impurities, while the soluble PRP is recovered in the permeate fraction. In the state of the art processes, the separation of the precipitate is carried out by centrifugation.

Dialysis is performed with 5 diavolumes of 40% (v/v) ethanol in 50 mM NaCl. The combination of reagents in this step is also an innovation in the process of the present invention, considering that the absence of salt makes microfiltration unfeasible, causing the PRP to be retained by the membrane. The fraction permeated in the membrane (2MF022) contains the PRP.

### 2.5 Second ultrafiltration, [2UF]

The ethanol concentrations in the steps [2MF] and [2UF] are different, in view of the stability of the membranes and the retention of the PRP. Therefore, after performing the microfiltration [2MF], the fraction 2MF022 must be diluted 1:1 with water, so that the ethanol concentration drops to 20% (v/v).

This dilution has not been used in any other process disclosed in the state of the art. However, it proved to be fundamental for the success of the PRP concentration in the [2UF] step.

After dilution, the sample is concentrated by ultrafiltration using a nominal molecular cut membrane of 100 kDa.

After concentration 10 to 20 times, dialysis is performed with 20% (v/v) ethanol in water. The concentrated fraction containing the PRP is referred to as 2UF 100.

### 2.6 Second precipitation with 60% ethanol

The second precipitation step aims to obtain the PRP in the precipitated phase. For this purpose, ethanol (90 - 99.5%) and sodium acetate (30%, pH 5.8) are added to the 2UF 100 fraction, so that the final concentration is 60% (v/v) ethanol and sodium acetate 2, 8% (m/v). Considering only the aqueous phase, the concentration of sodium acetate is 7%.

The contents should be stirred at room temperature for up to 4 hours. After this period, stirring is stopped and the PRP spontaneously decanted.

Decantation lasts up to 1 hour and then the supernatant is removed by pumping. The solubilization of the precipitate is carried out in PBS buffer pH 6.3, the content being homogenized from 2 to 16h at room temperature until complete solubilization. The solubilized fraction is referred to as SOLUB.

### 2.7 Third microfiltration, [3MF]

The SOLUB fraction is again subjected to microfiltration with a membrane with pores with a diameter of 0.22 µm.

Again, the objective of the process of the present invention is the retention of precipitated impurities, while the soluble PRP is recovered.

Dialysis is performed at constant volume with 5 - 15 diavolumes of PBS pH 6.3. The success of this step depends on the initial concentration of PRP, which should preferably be less than 2.4 g/L. In addition, the flow of the microfiltrate must be strictly controlled.

The fraction permeated in the membrane (3MF022) contains the PRP and proceeds to the final processing.

### 2.8 Third ultrafiltration, [3UF]

The last processing step is the concentration and dialysis in a nominal molecular cut membrane of 100 kDa.

The fraction 3MF022 is concentrated 5 to 15 times and then dialyzed at constant volume with 10 diavolumes of water. The concentrated fraction is referred to as 3UF 100, or simply "purified PRP".

This fraction can be frozen at temperatures below -18°C or lyophilized for further processing, in order to formulate the vaccine. Later steps may comprise the conjugation of PRP with proteins for the final formulation of the vaccine.

### DIFFERENT ASPECTS OF THE PRESENT INVENTION

### Use of Tangential Microfiltration

In the process developed by the present invention, it was found that the use of tangential microfiltration was essential in obtaining the required purity of PRP in relation to proteins and nucleic acids, namely, greater than 100 g of PRP per gram of nucleic acids (PR_{AN}) and more than 100 g of PRP per gram of protein (PR_{Prot}).

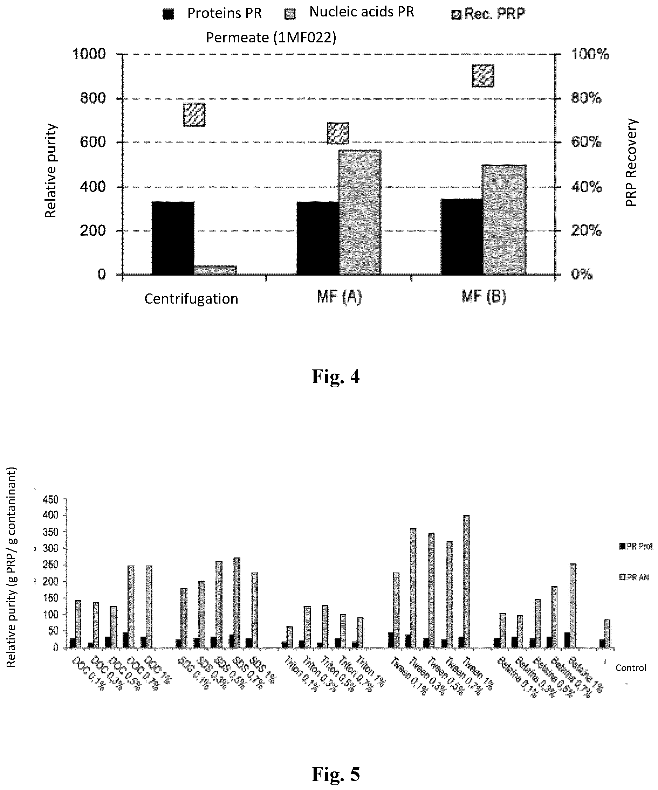

In Figure 4, three complete purification processes are compared, which differed only in the cell separation step. In the first, where centrifugation was used, the desired PRAN purity was not achieved. In the two other examples, where tangential microfiltration was used for cell separation, the relative purities PR_{AN} and PR_{Prot} were achieved and greatly exceeded.

### Addition of SDS in the first 100 kDa ultrafiltration

The PRP purification processes described in the state of the art generally employ a surfactant (CTAB or DOC) to promote PRP precipitation.

In the process disclosed by the present invention, another surfactant is used, namely, sodium dodecyl sulfate (SDS). The purpose of the addition, in this case, is to precipitate impurities and assist in microfiltration [1MF].

In the process described by the present invention, the SDS is added in the first step of ultrafiltration with a 100 kDa membrane. The surfactant was chosen by analyzing the *in vitro* precipitation of the fermented broth by Hib with 30% ethanol (plus 5% sodium acetate) and then 80% ethanol (plus 7% sodium acetate). In this experiment, the surfactant was added in the first precipitation. Among the surfactants DOC, SDS, Triton X-100, Tween 80 and betaine, SDS and Tween 80 resulted in the highest relative purities in relation to proteins and nucleic acids (Figure 5).

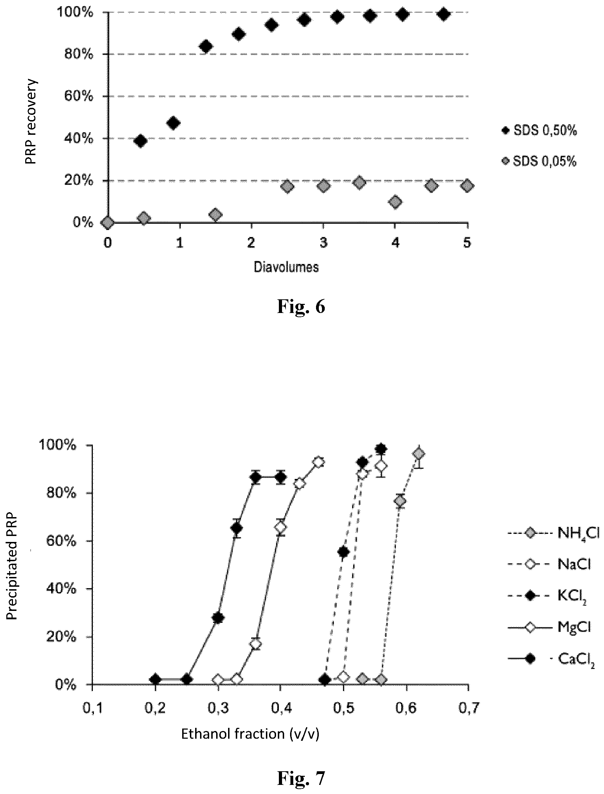

In addition to assisting in the precipitation of impurities, it was found that the SDS also promotes greater removal of proteins and nucleic acids during the first ultrafiltration, in addition to being a powerful auxiliary in the second microfiltration with 40% ethanol, increasing the flow of filtrate and PRP permeability (Figure 6).

As can be seen in Figure 6, the lower concentration of SDS made it impossible to recover the PRP under the conditions tested, due to the high retention of the PRP by the membrane in the absence or in the presence of low concentrations of SDS. Therefore, we believe that the addition of SDS during the process characterizes an innovation that is not an obvious combination of the processes already described in the state of the art.

### Change in ethanol concentration during precipitation

A technique commonly used in the precipitation of polysaccharides is the precipitation with ethanol in two steps with different concentrations of the solvent.

In the case of the PRP purification process described in the prior art documents, the concentration of ethanol in the first and second precipitation are 30 and 80%, respectively.

These concentrations are effective in the precipitation of impurities (in the first step) or in the PRP (in the second step), but result in great use of the organic solvent and significant losses of PRP during tangential filtration steps.

Furthermore, precipitation in these conditions is not sufficient to obtain the desired purity, requiring an enzymatic hydrolysis step of proteins and nucleic acids.

*In vitro* precipitation studies of purified PRP have shown that PRP precipitation is dependent on the presence of salts. Under these conditions, precipitation occurs abruptly with increasing solvent concentration (Figure 7).

This behavior has not been observed previously. A practical application of this behavior is that the concentrations of ethanol used in the purification of PRP can be changed. This change is not commonly used. For example, according to the state of the art, polysaccharide precipitation is usually carried out with ethanol concentrations above 70% and is unlikely to be optimized.

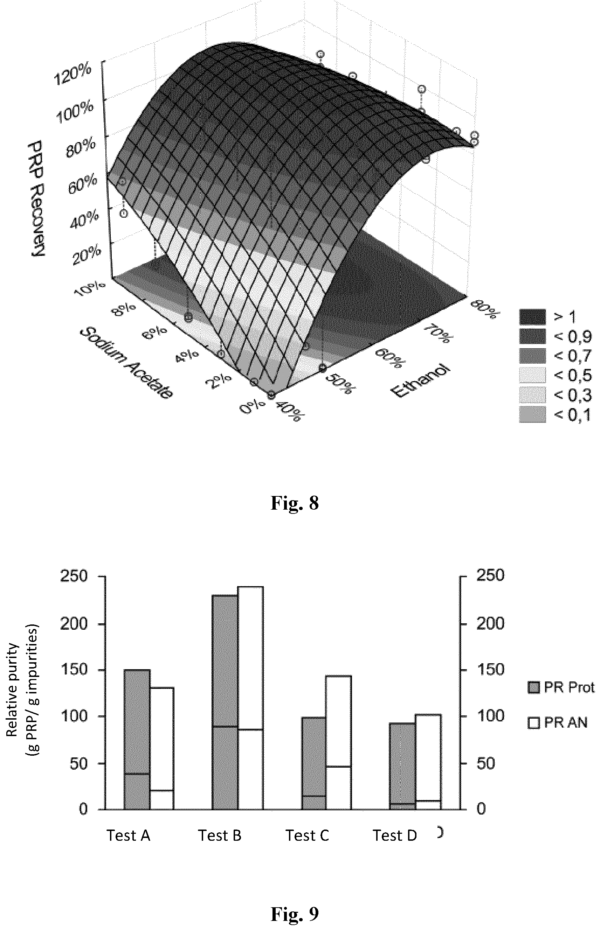

Changing the concentration of ethanol is a fundamental point in the process developed in the present invention. For this purpose, a study was carried out to verify the best condition for precipitation in both steps, by combining the reagents ethanol and sodium acetate (Figure 8).

From this study it was proved that, in fact, the PRP precipitation occurs in a narrow range of ethanol concentration in the presence of sodium acetate.

Together with the product purity data, a new condition was established for the PRP precipitation: 40% ethanol in the first step and 60% ethanol plus 7% sodium acetate in the second precipitation step.

Figure 8 shows the recovery of PRP in the supernatant phase after the second precipitation.

### Efficiency of the process of the present invention

The efficiency of the process of the present invention was compared to the traditional methodology through the sequential precipitation of the fermented broth under the conditions described previously.

The procedure was repeated four times. It was found that in all tests, the purity achieved with the process of the present invention was superior to the control condition (Figure 9).

Figure 9 shows PR_{Prot} (dark gray) and PR_{AN} (light gray) in the purification of different PRP batches. The black lines crossing the bars indicate the respective values found in the control. Control condition: 30% ethanol and 5% sodium acetate in the first precipitation; 80% ethanol and 7% sodium acetate in the second precipitation. Optimized condition: 40% ethanol in the first precipitation; 60% ethanol and 7% sodium acetate in the second precipitation.

The change in ethanol concentrations resulted in difficulties in the tangential filtration steps, due to the change in the membrane structure, which resulted in significant losses of PRP. For example, tangential ultrafiltration with 100 kDa pore membranes in the presence of 40% ethanol was successfully performed on the bench scale using 0.1 m² membranes.

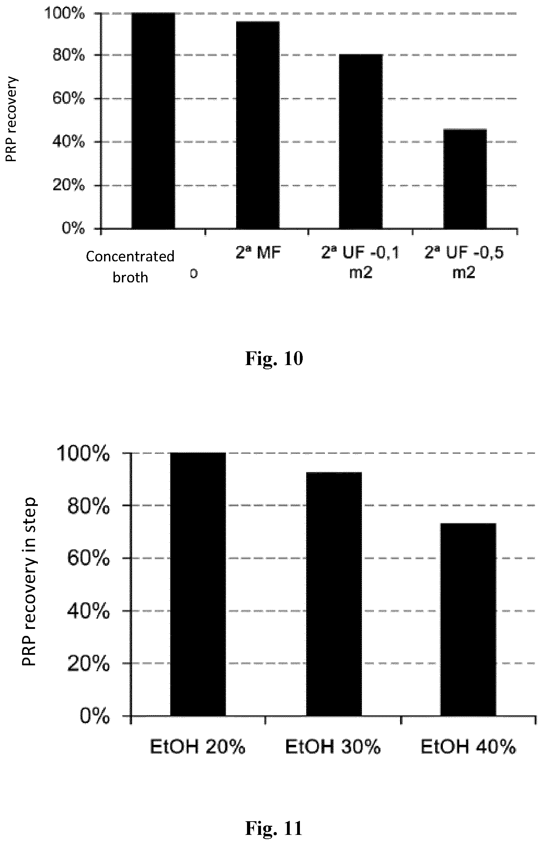

However, when staggering the process for the use of 0.5 m² membranes (dimensions of the cassette 17.8 x 21 cm) the losses of PRP were greater than 50%, which would make the process unfeasible (Figure 10).

Figure 10 shows a problem with staggering. Tangential ultrafiltration in a 100 kDa pore membrane. Solvent 40% ethanol in water.

To solve the staggering problem, a dilution step of the material to be ultrafiltered was added, so that the ethanol concentration was equal to 20%. This concentration was determined according to the verification of the losses of PRP in this step as a consequence of the concentration of ethanol used (Figure 11).

Figure 11 shows the effect of ethanol concentration in the second step of tangential ultrafiltration in a 100 kDa pore membrane. 0.5 m² membrane.

Another consequence of the change in the concentration of ethanol is the increased retention of PRP by microfiltration membranes, resulting in losses of PRP.

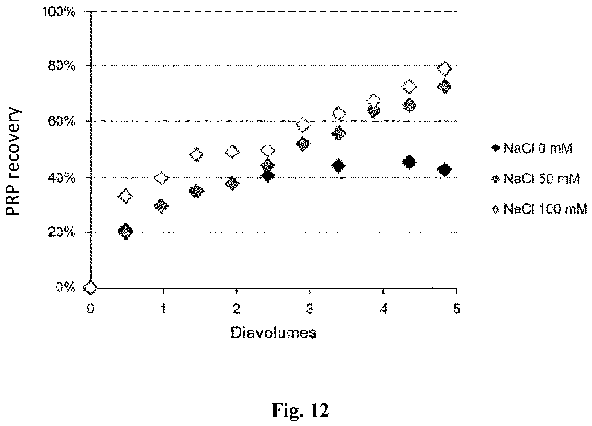

The solution to this problem occurred by increasing the saline concentration in the dialysis buffer (Figure 12).

However, due to the correlation between high salt concentration and loss of PRP in the later step, it was found that moderate concentrations of NaCl (50 mM) during the second 0.22 µm tangential microfiltration result in high product recoveries in both microfiltration and posterior ultrafiltration.

Figure 12 shows the effect of salt concentration in the aqueous fraction of the 40% Ethanol solution in the second microfiltration step of the PRP purification process.

Thus, although only some embodiments of the present invention have been shown, it will be understood that several omissions, substitutions and changes in the process for the production, recovery and purification of capsular polyribosyl-ribitol-phosphate (PRP), can be done by a person skilled in the art, without departing from the spirit and scope of the present invention.

It is expressly provided that all combinations of elements that perform the same function in substantially the same way to achieve the same results are within the scope of the invention. Substitutions of elements from one embodiment described to another are also fully intended and contemplated.

It is also necessary to understand that the drawings are not necessarily in scale, but that they are only of a conceptual nature. The intention is, therefore, to be limited, as indicated by the scope of the attached claims.

### Referencies

Ada, G., & Isaacs, D. (2003). Carbohydrate-protein conjugate vaccines. Clinical Micro-biology Infection, 9(2), 79-85.

Ali, T. R; Kroll, S; Lanfford, P. R. Haemophilus influenzae Microarrays: Virulence and Vaccines. Com Fubc. Genom. V. 3, p. 358-361, 2002;

Albani, S. M. F., da Silva, M. R., Takagi, M., & Cabrera-Crespo, J. (2012). Improvement in the purification process of the capsular polysaccharide from Haemophilus influenzae type b by using tangential ultrafiltration and diafiltration. Applied Biochemistry and Biotechnology, 167(7), 2068-2075.

Bisgard, K. M., Kao, A., Leake, J., Strebel, P. M., Perkins, B. a., & Wharton, M. (1998).Haemophilus influenzae invasive disease in the United States, 1994-1995: Near disappearance of a vaccine-preventable childhood disease. Emerging Infectious Diseases, 4(2), 229-237.

Bradley, T. D., & Mitchell, J. R. (1988). The determination of the kinetics of polysaccharide thermal degradation using high temperature viscosity measurements. Carbohydrate Polymers, 9(4), 257-267.

Crisel, R. M; Baker, R. S.; Dorman, D. E.Capsular polymer of Haemophilus influenzae, type b. I. Structural characterization of the capsular polymer of strain Eagan. J. Biol. Chen., v. 250, p. 4926-4930, 1975;
Egan, W., Schneerson, R., Werner, K. E., & Zon, G. (1982). Structural studies and chemistry of bacterial capsular polysaccharides. Investigations of phosphodiester-linked capsular polysaccharides isolated from Haemophilus influenzae types a, b, c, and f: NMR spectroscopic identification and chemical modification. Journal of the American Chemical Society, 104(10), 2898-2910.

GAVI. (2013). Hib vaccine. Retrieved from http://www.gavialliance.org/support/nvs/hib/

Lee H. Harrison, Vera Simonsen, Eliseu Alves Waldman. Emergence and disappearance of a virulent clone of Haemophilus influenzae biogroup aegyptius, cause of Brazilian purpuric fever, published by Clinical microbiology reviews 2008. DOI:10.1128/cmr.00020-08

Kelly DF1, Moxon ER, Pollard AJ . Haemophilus influenzae type b conjugate vaccines. Department of Paediatrics, John Radcliffe Hospital, University of Oxford, Headington, Oxford, UK Immunology. 2004 Oct; 113(2): 163-174. doi: 10.1111/j.1365-2567.2004.01971.x

Li, Y., & Breaker, R. R. (1999). Kinetics of RNA degradation by specific base catalysis of transesterification involving the 2_-hydroxyl group. Journal of the American Chemical Society, 121(23), 5364-5372.

Merritt, J., Allard, G., O'Toole, L., Swartz, R., & Licari, P. (2000). Development and scale-up of a fed-batch process for the production of capsular polysaccharide from Haemophilus influenzae. Journal of Biotechnology, 81(2-3),189-197.

Meunier, D. M. (1997). Molecular weight determinations. In F. A. Settle (Ed.), Hand-book of instrumental techniques for analytical chemistry (pp. 853-866). UpperSaddle River: Prentice-Hall.Stepto, R. F. T. (2009). Dispersity in polymer science (IUPAC recommendations 2009). Pure and Applied Chemistry, 81(2), 351-353.

Sturgess, A. W., Rush, K., Charbonneau, R. J., Lee, J. I., West, D. J., Sitrin, R. D.,et al. (1999). Haemophilus influenzae type b conjugate vaccine stability: Cat-alytic depolymerization of PRP in the presence of aluminum hydroxide. Vaccine, 17(9-10), 1169-1178.

ST. GEME III, J. W; Cutter D. Influence of pili, fibrils, and capsule on in vitro adherence by Haemophilus influenzae type b. Mol Microbiol. V. 1 n. 1, p 21-31, 1996.

Pittman M. Variation and type specificity in the bacterial species Haemophilus influenzae. J Exp Med 1931; 53(2): 471-93.

Takagi, M., Cabrera-Crespo, J., Zangirolami, T. C., Raw, I., & Tanizaki, M. M. (2006). Improved cultivation conditions for polysaccharide production by H. influenzae type b. Journal of Chemical Technology & Biotechnology, 81(2), 182-188.

Takagi, M., Lima, R. B., Albani, S. M. F., Zangirolami, T. C., Tanizaki, M. M., &Cabrera-Crespo, J. (2008). Purification of capsular polysaccharide produced by Haemophilus influenzae type b through a simple, efficient and suitable method forscale-up. Journal of Industrial Microbiology & Biotechnology, 35(11), 1217-1222.

UNICEF. (2013). Vaccine Price Data. Retrieved from http://www.unicef.org/supply/index 57476. html

WHO. (2013). Immunization surveillance, assessment and monitoring. Retrieved fromhttp://www.who.int/immunizationmonitoring/diseases/Hib/en/index.html.

Wilfert, C. M. (1990). Epidemiology of *Haemophilus influenzae* type b infections.

## Claims

1. Process for the cultivation of *Haemophilus influenzae* type b for the production of PRP **characterized by** comprising the following steps:
a) preparing batches from lyophilisate of the strain of *Haemophilus influenzae* type b GB3291, producer of the capsular polysaccharide Polyribosyl-Ribitol-Phosphate (PRP);
b) distributing the bacterial suspension in cryovials;
c) preparing a pre-inoculum from the bacterial suspension of the batch stored in liquid nitrogen;
d) transferring a volume of the bacterial suspension to a flask containing 50 ml of complex medium containing peptones of plant origin and yeast extract.
e) keeping the sample static at 37°C for 7 - 10 h in an anaerobic chamber;
f) transferring the sample to the 2 L inoculum flask, in an adequate volume so that 400 mL of the resulting suspension presents DO540 equal to 0.025;
g) incubating and stirring the suspension at 37°C "overnight";
h) transferring the appropriate suspension volume so that the initial DO540 of the initial culture medium, present in the bioreactor, is 0.1;
i) finishing the process after 20 hours of cultivation with DO540 around 20;
j) after completion of the cultivation, growth inhibitor is added to a final concentration of 0.2% and then the polysaccharide purification process begins.

2. Process, according to claim 1, **characterized in that** the bacterial suspension is distributed in cryovials, preferably of 1 mL, frozen at -20°C for 12 hours and then maintained until the moment of use in liquid nitrogen.

3. Process, according to claim 1, **characterized in that** after step e) the optical density of the sample is measured at 540 nm (DO540), and in step g) the suspension is stirred in an orbital shaker at 250 RPM and 37°C for at least 12 hours, until DO540 reaches between 5 and 7, and in step j) the growth inhibitor is selected from the group of sodium azide, Timerosal (C₉H₉HgNaO₂S), preferably, the growth inhibitor is sodium azide, and, wherein the bacterial cultivation is carried out in a simple batch regime until the total depletion of the glucose in the medium occurs, and wherein **in that** point the batch phase begins, fed by the addition of feeding medium at constant flow.

4. Process, according to claim 1, **characterized in that** the initial culture medium comprises:
10 g/l of soytone;
5.0 g/l of UF yeast extract;
5.0 g/l of glucose;
5.0 g/l of NaCl;
2.5 g/l of K₂HPO₄;
10 g/l of Na₂HPO₄;
5.8 g/l of NaH₂PO₄·H₂O;
0.015 g/l of NAD; and
0.03 g/1 of Hemine.

5. Process, according to claim 4, **characterized in that** the feed medium comprises:
10 g/l of soytone;
100.0 g/l of UF yeast extract;
200.0 g/l of glucose;
5.0 g/l of NaCl;
2.5 g/l of K₂HPO₄;
10 g/l of Na₂HPO₄;
5.8 g/l of NaH₂PO₄-H₂O;
0.015 g/l of NAD; and
0.03 g/1 of Hemine.

6. Process for the clarification and purification of PRP as defined in any of claims 1 to 5 **characterized in that** the process comprises the following steps:
a) 1^{st} microfiltration of the fermented broth using 0.22 µm pore membranes and PBS, pH 6.3 to obtain the permeate 1MF022;
b) The permeate is subjected to a 1^{st} ultrafiltration using a nominal molecular cut membrane of 100 kDa, 0.5% SDS in PBS, pH 6.3 to obtain the 1UF100 concentrate;
c) The 1UF100 concentrate is subjected to a 1^{st} precipitation by the addition of ethanol (95 - 99.5%). The final concentration of ethanol is 40% (v/v);
d) After the 1^{st} precipitation, the 2^{nd} microfiltration [2MF] is carried out with a membrane having pores with a diameter of 0.22 µm to obtain the permeate 2MF022;
e) Dilution of the fraction 2MF022 to 1:1 with water so that the ethanol concentration drops to 20% (v/v);
f) After dilution, the sample is concentrated by a 2^{nd} ultrafiltration using a nominal molecular cut membrane of 100 kDa to obtain the 2UF 100 concentrate;
g) After the concentration of 10 to 20 times, dialysis is carried out with ethanol 20% (v/v) in water;
h) The 2UF100 concentrate is subjected to a second precipitation with 60% ethanol and 2.8% sodium acetate (7% in the aqueous phase) and the contents are stirred at room temperature for up to 4 h;
i) After this period, the stirring is stopped and the PRP spontaneously decants;
j) The liquid phase containing 60% ethanol is transferred by pumping and the precipitate is subjected to a solubilization in PBS buffer pH 6.3, the content being homogenized from 2 to 16h at room temperature;
k) The solubilized product (SOLUB) is submitted to a 3^{rd} microfiltration [3MF] with a membrane with a pore diameter equal to 0.22 µm, wherein soluble PRP is recovered 3MF022;
l) The fraction permeated in the 3MF022 membrane is subjected to 3^{rd} ultrafiltration [3UF] with concentration and dialysis in a nominal molecular cut membrane of 100 kDa, wherein the fraction 3MF022 is concentrated 5 to 15 times and is then dialyzed at constant volume with 10 diavolumes of water to obtain concentrated fraction 3UF 100, or simply "purified PRP".

7. Process, according to claim 6, **characterized in that** the first microfiltration separates the bacterial cells and the PRP present in the extracellular medium referred to as "clarification", wherein the clarification of the fermented broth is carried out by tangential flow microfiltration , preferably, microfiltration is carried out with a membrane having pores with a diameter equal to 0.22 µm, and, during that step, the cells are dialyzed, preferably, the dialysis solution is PBS buffer pH 6.3 and, wherein the dialysis is carried out at constant volume with 10 - 25 diavolumes of buffer, wherein the permeate or microfiltered fraction 1MF022 contains the PRP, and the cells are present in the fraction retained by the membrane, which is discarded, wherein the fraction 1MF022 is subjected to concentration and ultrafiltration dialysis using a 100 kDa [1UF] nominal molecular cut membrane.

8. Process, according to claim 6, **characterized in that** the content is initially concentrated 15 to 45 times, and then dialyzed at constant volume with 6 diavolumes of PBS buffer pH 5.8 - 6.3 plus 0.5% SDS, wherein the concentrated fraction is called 1UF100, and, wherein with the addition of SDS, greater removal of impurities occurs during the first ultrafiltration, [1UF], and in the first precipitation step, as well as better performance of the second microfiltration step, [2MF].

9. Process, according to claim 6, **characterized in that** the first precipitation with ethanol is carried out by adding ethanol (95 - 99.5%) to the 1UF100 fraction, wherein the final concentration of ethanol is 40% (v/v), wherein the content is then kept under stirring for up to 16h at room temperature for the precipitation of impurities, mainly proteins and nucleic acids, while the PRP remains soluble, preferably, the precipitated content of the previous step is, then, subjected to the microfiltration with membrane having pores with a diameter of 0.22 µm [2MF] for the retention of precipitated impurities, while soluble PRP is recovered in the permeate fraction, wherein dialysis is carried out with 5 diavolumes of 40% ethanol (v/v) in 50 mM NaCl, and wherein the fraction permeated in the membrane 2MF022 contains the PRP.

10. Process, according to claim 9, **characterized in that** the concentrations of ethanol in steps [2MF] and [2UF] are different, in view of the stability of the membranes and the retention of the PRP, wherein after the completion of the microfiltration [2MF], the fraction 2MF022 should be diluted 1:1 with water, so that the ethanol concentration decreases to 20% (v/v), and, wherein after dilution, the sample is concentrated by ultrafiltration using a nominal molecular cut membrane of 100 kDa, wherein after the concentration of 10 to 20 times, dialysis with 20% ethanol (v/v) in water is carried out and the concentrated fraction containing the PRP is referred to as 2UF 100.

11. Process, according to claim 10, **characterized in that** the second precipitation step obtains the PRP in the solid phase, wherein, for this purpose, ethanol (90 - 99.5%) and sodium acetate (30%, pH 5.8) are added to the 2UF100 fraction, so that the final concentration is 60% ethanol (v/v) and sodium acetate 2.8% (w/v) (equivalent to 7% in the aqueous phase), wherein the content is stirred at room temperature for up to 4 hours, wherein after that period the stirring is stopped and the PRP decants spontaneously, preferably, the decantation lasts up to 1 hour and then the supernatant is removed by pumping, and the solubilization of the precipitate is carried out in PBS buffer pH 6.3, the content being homogenized from 2 to 16h at room temperature, and the solubilized fraction is referred to as SOLUB, preferably, the SOLUB fraction is again subjected to microfiltration in membrane having pores with a diameter of 0.22 µm [3MF] retaining precipitated impurities, while soluble PRP is recovered, and dialysis is carried out at constant volume with 5 - 15 diavolumes of PBS pH 6.3.

12. Process, according to claim 11, **characterized in that** the initial concentration of PRP should be less than 3.6 g/L, preferably less than 2.4 g/L. and the microfiltrate flow should be strictly controlled, the fraction permeated in the 3MF022 membrane contains the PRP, preferably, the fraction 3MF022 is concentrated 5 to 15 times and then dialyzed at constant volume with 10 diavolumes of water, wherein the concentrated fraction is referred to as 3UF 100, or simply "purified PRP", wherein this fraction can be frozen at temperatures below -18°C or lyophilized for further processing.

13. Use of the purified PRP obtained by the process as defined in any of claims 1 to 12 **characterized in that** it is for the preparation of vaccine against *H. influenzae* type b.

14. Use, according to claim 13, **characterized in that** the vaccine is a pentavalent vaccine, composed by five antigens: DTP (diphtheria-tetanus-pertussis), HepB (hepatitis B) and Hib.

15. Use of the purified PRP obtained by the process as defined in any of claims 1 to 12 **characterized in that** it is for the conjugation of PRP with proteins.
